(19) Europäisches Patentamt
European Patent Office
Office européen des brevets

(11) EP 3 331 849 B1

(12) EUROPEAN PATENT SPECIFICATION

(45) Date of publication and mention of the grant of the patent:
**26.12.2018 Bulletin 2018/52**

(21) Application number: **16750724.3**

(22) Date of filing: **03.08.2016**

(51) Int Cl.:
***C07C 227/36*** *(2006.01)* ***C07C 229/48*** *(2006.01)*

(86) International application number:
**PCT/EP2016/068534**

(87) International publication number:
**WO 2017/021445 (09.02.2017 Gazette 2017/06)**

(54) **TILIDINE ISOMERIZATION AND CRYSTALLIZATION PROCESS**

TILIDINISOMERISATIONS- UND KRISTALLISATIONSVERFAHREN

ISOMÉRISATION DE TILIDINE ET PROCÉDÉ DE CRISTALLISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2015 EP 15179979**

(43) Date of publication of application:
**13.06.2018 Bulletin 2018/24**

(73) Proprietor: **Siegfried AG**
**4800 Zofingen (CH)**

(72) Inventors:
- **BAHR, Nicolaus**
  **32120 Hiddenhausen (DE)**
- **SCHÄFER, Bernd**
  **76889 Dierbach (DE)**
- **SIEGEL, Wolfgang**
  **67117 Limburgerhof (DE)**

(74) Representative: **Harms, Guido**
**Siegfried Hameln Services GmbH**
**Langes Feld 13**
**31789 Hameln (DE)**

(56) References cited:
**WO-A2-99/03821 GB-A- 1 226 318**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 331 849 B1

## Description

Ethyl (E)-2-(dimethylamino)-1-phenylcyclohex-3-ene-1-carboxylate, i.e. trans-tilidine, is a potent opioid analgesic.

The structure, synthesis and activity of trans-tilidine have been described by Gödecke (DE 1 518 959, DE 1 618 476, DE 1 618 482, DE 1 668 156, DE 1 768 704, DE 1 811 808, DE 1 923 619, DE 1 923 620, K.-O. Vollmer, F. W. Koss, Arzneimittelforschung 20 (1970) 990; G. Satzinger, Liebigs Ann. Chem. 728 (1969) 64; G. Satzinger, Liebigs Ann. Chem. 758 (1972) 43; G. Satzinger, Liebigs Ann. Chem. 758 (1972) 65) and at about the same time by Warner-Lambert (GB 1 120 186, US 3,637,806).

Tilidine is usually obtained by reacting the enamine formed from Crotonaldehyde and dimethyl amine with ethyl 2-phenylacrylate in a Diels-Alder reaction. This provides a mixture of about 60 % of cis-tilidine (ethyl (Z)-2-(dimethylamino)-1-phenylcyclohex-3-ene-1-carboxylate) and about 40 % of trans-tildine (ethyl (E)-2-(dimethylamino)-1-phenylcyclohex-3-ene-1-carboxylate) (DE 1 923 620).

As trans-tilidine is more effective than cis-tilidine, processes were developed for separating trans-tilidine from cis-tilidine and for converting cis-tilidine into trans-tilidine.

DE 1 618 482 relates to a process for converting cis-tilidine into trans-tilidine by keeping it for several hours at about 150 °C to 160 °C under an inert gas in the presence of 0.3 to 1 % by weight of a disubstituted amine having a boiling temperature in the range from about 150 °C to 160 °C. This results in an isomerization product comprising 25 to 30 % by weight of trans-tilidine.

DE 1 951 587 teaches that trans-tilidine may be prepared from cis-tilidine by heating cis-tilidine in an acidic amphiprotic solvent to 90 °C to 120 °C.

GB 1 226 318 describes a process for separating trans-tilidine by treating an isomeric mixture of trans-tilidine and cis-tildine with oxalic acid or fumaric acid in an organic solvent at a temperature up to reflux temperature for a period of time sufficient to dissolve the isomeric mixture and the acid in the solvent and recovering the acid salt of trans-tilidine which precipitates from the reaction mixture. The same document describes a process for the partial isomerization of cis-tilidine which comprises heating cis-tilidine with about 2 to 6 mols of an organic acid or an anhydride of the organic acid per mol of cis-tilidine at a temperature from about 60 °C to about 130 °C.

GB 1 226 318 fails to suggest an efficient integration of the isomerization and separation into one process. As shown, for example, at page 4, lines 85 to 103, the isomerization product is isolated in a tedious work-up procedure before the trans-tilidine is separated. This work-up procedure involves removing of solvent under vaccum on a steam bath, adding toluene, removing toluene under vacuum on a steam bath, adding water, adding aqueous potassium hydroxide, extracting the aqueous phase with toluene, washing the toluene phase with water, and removing the toluene from the toluene phase under vaccum on a steam bath. The work-up results in a cis-tilidine and trans-tilidine containing mixture. A separation procedure in which this solid mixture is once again treated with an organic solvent is then applied in order to separate trans-tildine in the form of trans-tilidine oxalate from this mixture.

Whenever alcohols such as, for example, 1-butanol are present in the isomerization procedure of GB 1 226 318, the isomerization is carried out in the essential absence of water. The present inventors have found that such isomerization conditions do favor the formation of transesterification side products that are difficult to separate. It is thus evident that a further disadvantage of GB 1 226 318 consists in the formation of isomerization side products that are difficult to separate.

The problem underlying the present invention is thus the provision of a process that allows for an efficient integration of both, isomerization and separation and which yields trans-tilidine in a form that is essentially free of side products which are difficult to separate.

The problem is solved by a tilidine isomerization and crystallization process, comprising

a) providing a reaction mixture comprising cis-tilidine, a dicarboxylic acid, water and an alcohol having 3 to 6 carbon atoms;

b) maintaining the reaction mixture at a temperature T of from 80 °C to 150° C for a period of time t, T and t meeting the following condition:

$$500 \leq k^{(T-80)} \cdot t$$

preferably $500 \leq k^{(T-80)} \cdot t \leq 5000$

more preferred $600 \leq k^{(T-80)} \cdot t \leq 2500$

wherein

k is 1,07738
T is the temperature in °C;
t is the time in minutes at the temperature T;

c) removing water from the reaction mixture and crystallizing a trans-tilidine dicarboxylic acid salt from the reaction mixture.

The removal of water in step c) is preferably carried out at a temperature of less than 80 °C.

The term "cis-tilidine" as used herein refers to a mixture of the enantiomers:

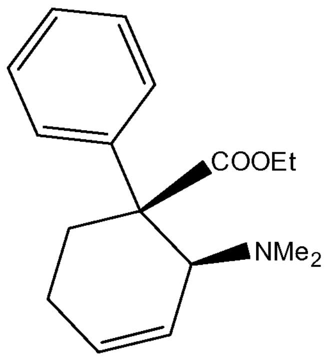

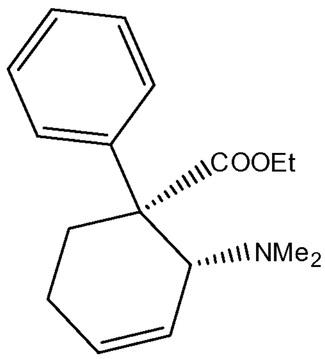

The term "trans-tilidine" as used herein refers to a mixture of the enantiomers:

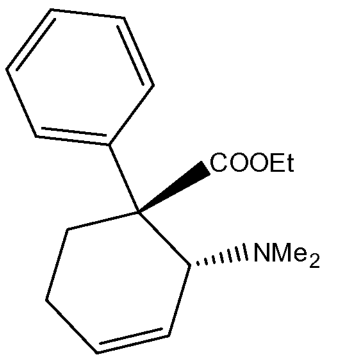

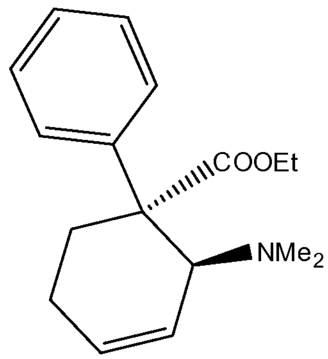

The term "tilidine" as used herein refers to any mixture of cis-tilidine and trans-tilidine.

The process according to the invention allows for efficient integration of both, isomerization and separation. The isomerization product, i.e. trans-tilidine, is crystallized directly from the reaction mixture in the form of its dicarboxylic acid salt without any intermediate tedious work-up. In a preferred process according to the invention, the crystallized trans-tilidine dicarboxylic acid salt may be separated from the mother liquor by an appropriate solid liquid phase separation method, for example, by filtration or centrifugation.

This process further yields trans-tilidine in a form that is essentially free of side products which are difficult to separate. Such side products are, in particular, tilidine derived compounds wherein the -O-ethyl group is replaced by the -O-alkyl group of the alcohol having 3 to 6 carbon atoms that is comprised by the reaction mixture, e.g., -O-(2-propyl) when the alcohol comprised by the reaction mixture is 2-propanol, e.g., 2-propyl 2-(dimethylamino)-1-phenylcyclohex-3-ene-1-carboxylate. Since such transesterification products are difficult to separate, it is pivotal to suppress their formation. The formation and co-crystallization of such side products is efficiently suppressed in the process according to the invention.

The presence of water in step b) and the essential absence of water after the removal of water in step c) are important for integrating isomerization and separation efficiently into the process, and for obtaining trans-tilidine in a form that is essentially free of side products which are difficult to separate. It has been found that the presence of water in step b) effectively suppresses the formation of tilidine transesterification side products wherein the -O-ethyl group is replaced by the -O-alkyl group of the alcohol comprised in the reaction mixture. The removal of water in step c) results in a reaction mixture that contains less water than the reaction mixture in steps a) and b) an that is preferably essentially free of water. This reduces the solubility of the trans-tilidine dicarboxylic acid salt in the reaction mixture and thus facilitates its crystallization.

The reaction mixture provided in step a) does comprise cis-tilidine. It may further comprise trans-tilidine. In general, the yield of trans-tilidine formed from cis-tilidine is high when there is an initial excess of cis-tilidine. In general, the reaction mixture provided in step a) does therefore comprise an excess of cis-tilidine over trans-tilidine, for example, at least 1.1 moles of cis-tilidine per mole of trans-tilidine, preferably at least 2 moles of cis-tilidine per mole of trans-tilidine, more preferably at least 5 moles of cis-tilidine per mole of trans-tilidine, e.g., 5 to 1000 moles of cis-tilidine per mole of trans-tilidine.

The isomerization can be carried out with a broad range of different overall tilidine contents in the reaction mixture. The overall tilidine content, i.e. the sum of cis-tilidine and trans-tilidine contents, in the reaction mixture provided in step a), is in general 5 to 80 % by weight of the reaction mixture, preferably 10 to 70 % by weight, more preferably 15 to 65 % by weight, further preferably 20 to 60 % by weight, most preferably 22 to 55 % by weight.

In step b), the reaction mixture may, for example, comprise 0.2 to 50 moles of water per mole of tilidine, preferably 0.5 to 20 moles of water per mole of tilidine, more preferably 0.8 to 15 moles of water per mole of tilidine, further preferably 1 to 10 moles of water per mole of tilidine, most preferably 1.5 to 7.5 moles of water per mole of tilidine. The molar amount of tilidine refers to the sum of the molar amounts of trans-tilidine and cis-tilidine. The exact water content of the reaction mixture in step b) is of minor importance. The water content in the reaction mixture is in general 1 to 25 % by weight of the reaction mixture, preferably 1.5 to 20 % by weight, more preferably 2 to 16 % by weight, most preferably 2.5 to 12 % by weight of the reaction mixture.

Any alcohol having 3 to 6 carbon atoms can be used. The alcohol having 3 to 6 carbon atoms is preferably a monohydric alcohol, i.e. an alcohol having one hydroxyl group. The alcohol is, for example, saturated and may be cyclic or acyclic, preferably acyclic. The alcohol is, for example, selected from 2-propanol, 2-butanol, tert-butanol, 2-pentanol, 3-pentanol, 2-methyl-2-butanol, 2,2-dimethyl-1-propanol, 2-hexanol, 2-methyl-2-pentanol, 3-hexanol, 3-methyl-3-pentanol, cyclohexanol and their mixtures. A particularly preferred alcohol having 3 to 6 carbon atoms is 2-propanol.

The alcohol having 3 to 6 carbon and the water together form a solvent that dissolves at least part of the tilidine and at least part of the dicarboxylic acid. In general, the total amount of the alcohol and water is chosen such that both, the tilidine and the dicarboxylic acid are fully dissolved in the reaction mixture at the highest temperature that is reached by the reaction mixture in step b), preferably such that both, the tilidine and the dicarboxylic acid are fully dissolved in the reaction mixture at 80 °C. The content of the alcohol in the reaction mixture in step b) is, for example, 10 to 85 % by weight of the reaction mixture, preferably 15 to 75 % by weight, more preferably 20 to 70 % by weight, most preferably 20 to 65 % by weight.

Many different dicarboxylic acids may be used in the process according to the invention. The usefulness of a specific dicarboxylic acid is determined by the solubility of its salts formed with trans-tilidine, and cis-tilidine, respectively. The dicarboxylic acid that is used in the process according to the invention forms a trans-tilidine dicarboxylic acid salt with relatively low solubility in the alcohol having 3 to 6 carbon atoms. It further forms no cis-tilidine dicarboxylic acid salt or a cis-tilidine dicarboxylic acid salt with relatively high solubility in the alcohol having 3 to 6 carbon atoms.

Any dicarboxylic acid is preferred, for which the solubility of the trans-tilidine dicarboxylic acid salt in the alcohol having 3 to 6 carbon atoms is at most 100 g/L at 20 °C, preferably at most 50 g/L at 20 °C, more preferably at most 25 g/L at 20 °C, most preferably at most 10 g/L at 20 °C, and for which the solubility of the cis-tilidine dicarboxylic acid salt in the alcohol having 3 to 6 carbon atoms is at least 3 times, preferably at least 10 times, e.g., at least 30 times, in particular at least 100 times higher than the solubility of the trans-tilidine dicarboxylic acid salt.

The dicarboxylic acid is preferably selected from aliphatic and aromatic dicarboxylic acids. Preferred dicarboxylic acids are fumaric acid and oxalic acid. A particularly preferred dicarboxylic acid is oxalic acid.

It is beneficial when the molar amount of tilidine comprised by the reaction mixture, i.e. the sum of the molar amounts of cis-tilidine and trans-tilidine, and the molar amount of the dicarboxylic acid comprised by the reaction mixture are in the same range. A high molar excess of dicarboxylic acid with regard to tilidine may require a relatively high amount of solvent in order to fully dissolve the dicarboxylic acid. A molar excess of tilidine with regard to dicarboxylic acid may, on the other hand, result in a decreased crystallization yield in step c), in particular when there is molar excess of trans-tilidine with regard to dicarboxylic acid. The reaction mixture may, for example, comprise 0.25 to 4 moles of dicarboxylic acid per mole of tilidine, preferably 0.3 to 3 moles of dicarboxylic acid per mole of tilidine, most preferably 0.5 to 2 moles of dicarboxylic acid per mole of tilidine, in particular 0.8 to 1.2 moles of dicarboxylic acid per mole of tilidine.

In step b), the reaction mixture is maintained at a temperature T of from 80 °C to 150 °C. At less than 80 °C the isomerization rate is too small. Temperatures of more than 150 °C lead to an increased formation of side products which are difficult to separate.

In general, the temperature T may vary within the range from 80 °C to 150 °C throughout step b). Preferably, the temperature T reaches a maximum $T^{max}$ of from 110 °C to 150 °C, for example, of from 130 °C to 150 °C, in particular of from 135 °C to 145 °C in step b).

The rates of the isomerization reaction and of undesired side reactions are temperature dependent. Long isomerization times are preferred only when relatively low isomerization temperatures are applied, whereas very short isomerization times are preferred only when relatively high isomerization temperatures are applied. The process according to the invention therefore requires that the temperature T of from 80° to 150° C is maintained for a period of time t, T and t meeting the following condition:

$$500 \leq k^{(T\text{-}80)} \cdot t$$

wherein

- k is 1,07738
- T is the temperature in °C;
- t is the time in minutes at the temperature T.

The constant k accounts for the increase of isomerization rate brought along by an increase of T by 1 °C.

As mentioned above, the temperature T may vary within the temperature range from 80 °C to 150 °C. At each temperature, the isomerization proceeds at a temperature dependent isomerization rate. In order to take the temperature variations and their influence on the isomerization rate into consideration, the above condition may be expanded into a sum, wherein the individual summands account for the time at a specific temperature within the temperature range:

$$500 \leq k^{(T_1\text{-}80)} \cdot \qquad + k^{(T_2\text{-}80)} \cdot \qquad + \ldots$$

wherein $t_1$ is the residence time of the reaction mixture at temperature $T_1$; $t_2$ is the residence time of the reaction mixture at temperature $T_2$; etc.

The reaction mixture is preferably heated quickly at the beginning of step b) from a temperature below 80 °C to a temperature in the range from 130 °C to 150 °C, e.g., 135 °C to 145 °C. Heating of the reaction mixture is, for example, carried out such, that the temperature increases by at least 0.15 Kelvin per second (K/s) in average, preferably at least 0.25 K/s in average, most preferably at least 0.4 K/s in average, in particular by 0.4 K/s to 10 K/s in average. The reaction mixture is kept at 130 °C to 150 °C, e.g., 135 °C to 145 °C and then cooled to a temperature of less than 80 °C. Cooling of the reaction mixture is, for example, carried out such, that its temperature decreases by at least 0.15 Kelvin per second (K/s) in average, preferably at least 0.25 K/s in average, most preferably at least 0.4 K/s in average, in particular by 0.4 K/s to 10 K/s in average.

Even though the process according to the invention can be carried out with any volume of reaction mixture, it is preferably carried out with large volumes. It has been pointed out above, that fast heating of the reaction mixture is preferred in order to further minimize the formation of side products that are difficult to separate. However, a careful but fast heating of larger volumes of reaction mixture, e.g., of intermediate milliliter to multi liter volumes of reaction mixture, is difficult to achieve in a discontinuous process. Fast heating in a reaction flask, e.g., a round-bottom flask, can lead to local overheating and therefore favor the formation of such side products. This is because the surface of the flask is relatively small, as compared to the volume of reaction mixture in the flask. Much heat has to be applied per unit of flask surface in order to ensure fast heating of the reaction mixture. This brings along the risk of local overheating.

It is easier to achieve a careful but fast heating of intermediate milliliter to multi liter volumes of reaction mixture in a continuous process. Step b) is thus preferably carried out continuously. The reaction mixture is directed into a reactor that has a relatively large reactor surface per reactor volume. When the ratio of heated reactor surface to reactor volume is large, fast heating of the reaction mixture can be achieved without local overheating because it is sufficient to supply a relatively small amount of heat per unit of reactor surface in order to achive fast heating. The ratio of heated reactor surface to reactor volume is, for example, at least 1.5 $cm^{-1}$, preferably at least 2 $cm^{-1}$, more preferably at least 3 $cm^{-1}$, most preferably at least 5 $cm^{-1}$.

Step b) is preferably carried out continuously in a plug flow type reactor. The plug flow type reactor may, for example, comprise a tube with a reactor inlet, an optional unheated section of the tube at the reactor inlet, a central heated section of the tube, an optional unheated section of the tube at the reactor outlet and a reactor outlet. The heated section of the tube may, for example, be surrounded by a heat source, such as a heating bath or a heating band, e.g., an electrical heating band, which delivers the heat to the heated section of the tube. The heated section of the tube may, for example, be located in a heating bath, i.e., an oil bath, with the inlet and the outlet of the reactor and the optional unheated sections of the tube sticking out of the heating bath. In general, the temperature of the surrounding heat source is constant along the heated tube. The reaction mixture entering the tube is heated very quickly as soon as it enters the heated section of the tube to a temperature close to the temperature of the surrounding heat source and remains essentially constant until the reaction mixture reaches the end of the heated section of the tube. The flow of the reaction mixture in step b) is preferably laminar.

Preferably, none of the components comprises by the reaction mixture provided in step a), i.e. the cis-tilidine, the dicarboxylic acid, the water, and the alcohol, is added to the reaction mixture or removed from the reaction mixture in step b). Alternatively, the reaction mixture may be supplemented with starting materials that are consumed in the time-course of step b). It is, for example, possible to feed a cis-tilidine comprising solution into one or multiple optional additional inlets of the plug flow type reactor that are connected to different sections of the heated section of the tube.

In step c), water is removed from the reaction mixture. In general, essentially all of the water comprised by the reaction mixture is removed before the trans-tilidine dicarboxylic acid salt is crystallized from the reaction mixture. The water content of the reaction mixture after removing water from the reaction mixture is, for example, 0 to 0.5 % by weight, preferably 0 to 0.2 % by weight, more preferably 0 to 0.1 % by weight, in particular 0 to 0.05 % by weight, e.g., 0 to 0.01 % by weight or 0 to 0.001 % by weight.

The water may be removed from the reaction mixture by any means suitable for removing water from reaction mixtures. Anhydrous magnesium sulfate or other drying agents known to the person skilled in the art may be added to the reaction mixture and after the water has been removed from the reaction mixture by the drying agent be separated from the reaction mixture by solid liquid phase separation, e.g., filtration or centrifugation.

The removal of water is preferably achieved by azeotropic distillation. Before the water is removed from the reaction mixture by azeotropic distillation, one or multiple compounds that form low boiling azeotropes with water, i.e. azeotropes that have a boiling temperature lower than the boiling temperature of the alcohol having 3 to 6 carbon atoms, may be added.

Preferably, the alcohol having 3 to 6 carbon atoms itself forms an azeotropic mixture with water and water is removed from the reaction mixture by azeotropic distillation. In this azeotropic distillation the water is removed from the reaction mixture in the form of an azeotropic mixture of the alcohol and the water. If the alcohol having 3 to 6 carbon atoms forms an azeotrope with water, there is no need for adding another compound that forms a low boiling azeotrope with water.

Preferably, the water is removed from the reaction mixture at a temperature of less than 80 °C. The azeotropic distillation is thus preferably carried out at reduced pressure, for example at less than 500 mbar, preferably less than 250 mbar, more preferably less than 100 mbar, in particular at less than 50 mbar.

All pressures indicated herein are absolute pressures.

The azeotropic distillation may be carried out continuously or discontinuously.

Any vaccuum distillation apparatus may be used for the discontinuous removal of water from the reaction mixture.

The continuous azeotropic distillation may, for example, be carried out by feeding the reaction mixture obtained from step b), e.g., the stream obtained from the outlet of the tube comprised by the plug flow type reactor, through an inlet of a distillation column. The distillation column is preferably operated at reduced pressure. The bottom of the distillation column is heated. The distillation column does preferably comprise a first outlet that is located above the inlet and a second outlet that is located below the inlet. The azeotropic mixture of the alcohol and water may, for example, be passed through the first outlet and the reaction mixture from which water has been removed may, for example be passed through the second outlet.

Additional alcohol having 3 to 6 carbon atoms may be added to the reaction mixture before the water is removed from the reaction mixture by means of azeotropic distillation in the form of an azeotropic mixture of the alcohol and the water. The reaction mixture may thus be diluted with alcohol having 3 to 6 carbon atoms before the water is removed by azeotropic distillation.

The crystallization of the trans-tilidine dicarboxylic acid salt from the reaction mixture may be carried out at a broad range of different contents of tilidine and dicarboxylic acid.

The content of tilidine, i.e. the sum of the contents of cis-tilidine and trans-tilidine is, for example, 5 to 40 % by weight of the reaction mixture obtained after the removal of the water, preferably 8 to 35 % by weight of the reaction mixture, more preferably 10 to 30 % by weight, most preferably 15 to 25 % by weight.

The content of dicarboxylic acid is, for example, 1 to 15 % by weight of the reaction mixture obtained after the removal of the water, preferably 2 to 13 % by weight, more preferably 3 to 11 % by weight, most preferably 5 to 9 % by weight.

These contents of tilidine and dicarboxylic acid are particularly preferred when the alcohol having 3 to 6 carbon atoms is 2-propanol and the dicarboxylic acid is oxalic acid. When another alcohol and/or another dicarboxylic acid are used, the solubility of the trans-tilidine dicarboxylic acid salt may be different. A person skilled in the art knows, how the solubility of a specific trans-tilidine dicarboxylic acid salt can be determined in a specific alcohol having 3 to 6 carbon atoms. The skilled person would adjust the crystallization conditions accordingly.

Preferably, the desired content of tilidine and dicarboxylic acid is adjusted directly when the water is removed from the reaction mixture, e.g. by azeotropic distillation wherein the water is removed from the reaction mixture in the form of an azeotropic mixture of the alcohol having 3 to 6 carbon atoms and the water. The amount of alcohol having 3 to 6 carbon atoms that is used for diluting the reaction mixture may be chosen accordingly. Alternatively, additional alcohol having 3 to 6 carbon atoms may be removed by distillation after the azeotropic mixture of water and the alcohol

has been removed.

The crystallization of the trans-tilidine dicarboxylic acid salt from the reaction mixture may, for example, comprise cooling of the reaction mixture to 25 °C or less.

A small amount of solid trans-tilidine dicarboxylic acid salt may be added in order to induce the crystallization of the trans-tilidine dicarboxylic acid salt from the reaction mixture.

The crystallized trans-tilidine dicarboxylic acid salt may, for example, be separated from the mother liquor by any sort of solid liquid phase separation. Preferably, the solid liquid phase separation is selected from filtration or centrifugation, most preferably filtration.

The separated crystallized trans-tilidine dicarboxylic acid salt may be treated in order to remove at least part of the impurities comprised by the crystallized trans-tilidine dicarboxylic acid salt. This is preferably achieved by washing. The crystallized trans-tilidine dicarboxylic acid salt is preferably washed with additional cold solvent, for example, with additional cold alcohol having 3 to 6 carbon atoms. Preferably, the structure of the additional alcohol used for washing is the same as the structure of the alcohol comprised by the reaction mixture provided in step a).

The mother liquor is preferably recycled into step a). The mother liquor is preferably concentrated, e.g., by evaporation of some of the alcohol having 3 to 6 carbon atoms comprised by the mother liquor, for example, by distilling off some of the alcohol, before it is recycled into step a). Before the mother liquor is concentrated, it may be combined with the solvent that has been used for washing the crystallized trans-tilidine dicarboxylic acid salt.

Recycling of the mother liquor into step a) means that the reaction mixture provided in step a) can at least in part consist of recycled mother liquor. In general, the recycled mother liquor does not comprise any water. It is thus preferred to add water to the recycled mother liquor. Furthermore, some dicarboxylic acid is preferably added to the recycled mother liquor in order to increase the dicarboxylic acid content to a desired level.

The invention is illustrated in detail by the examples which follow.

## Examples

### Example 1

Batch Isomerization in the presence of water

a) Provision of reaction mixture

2950 g of a liquid containing 12.9 % of tilidine (1270 mmol; 92.3 % cis-tilidine, 5.6 % trans-tilidine) in 2-propanol was concentrated in a rotary evaporator at 100 mbar such that a solution containing 29.4 % of tilidine was obtained. 116.7 g (1270 mmol) of oxalic acid was added and another 666 g of solvent were removed at 100 mbar before 45.7 g (2540 mmol) of water were added.

b) Isomerization reaction

The reaction mixture obtained in step a) was heated to 90 °C and kept at this temperature for 9h.

c) Water removal and crystallization

After cooling down to 50 °C, 1671 g of 2-propanol were added dropwise and 658 g of solvent were then removed by distillation at 100 mbar. 0.1 g of trans-tilidine oxalate were added, the mixture was cooled to 20 °C and then filtered. The filtration provided a filter cake and a mother liquor. The filter cake was washed twice with 381 g of 2-propanol. The washed filter cake was then dried in a vacuum drying oven at 50 °C.

220.9 g of trans-tilidine oxalate were obtained (570 mmol, purity: 93.8 % by weight, yield: 45 % based on the total initial amount of tilidine). No 2-propyl 2-(dimethylamino)-1-phenylcyclohex-3-ene-1-carboxylate side product could be detected in the product.

### Example 2

Batch Isomerization in the presence of water

a) Provision of reaction mixture

To the combined mother and wash liquors obtained in example 1, step c) (2029 g, 9.6 % of tilidine, 538 mmol tilidine, 445 mmol oxalic acid) 8.5 g of oxalic acid (93 mmol) were added. 1650 g of solvent were distilled off and then

48.5 g of water (2690 mmol) were added.

b) Isomerization reaction

The reaction mixture obtained in step a) was heated to 90 °C and kept at this temperature for 9h.

c) Water removal and crystallization

After cooling down to 50 °C, 947 g of 2-propanol were added dropwise and 696 g of solvent were then removed by distillation at 100 mbar. The mixture was cooled to 30 °C. 0.1 g of trans-Tildine oxalate were added, the mixture was cooled further to 0 °C and then filtered. The filtration provided a filter cake and a mother liquor. The filter cake was washed twice with 269 g of 2-propanol. The washed filter cake was then dried in a vacuum drying oven at 50 °C.

95.8 g of trans-tilidine oxalate were obtained (227 mmol, purity: 85.9 % by weight, yield: 42 % based on the total initial amount of tilidine used in Example 2, step a). The product comprised 0.106 % by weight of 2-propyl 2-(dimethylamino)-1-phenylcyclohex-3-ene-1-carboxylate side product.

Comparative example 3

Batch Isomerization in the absence of water

Example 1 was repeated without adding water. The product comprised 0.086 % by weight of 2-propyl 2-(dimethylamino)-1-phenylcyclohex-3-ene-1-carboxylate side product.

Comparative example 4

Batch Isomerization in the absence of water

Example 2 was repeated without adding water. The product comprised 0.247 % by weight of 2-propyl 2-(dimethylamino)-1-phenylcyclohex-3-ene-1-carboxylate side product.

Example 5

a) Provision of reaction mixture

5793 g of a liquid containing tilidine (447,8 g, 1638 mmol; 92.7 % cis-tilidine, 5.5 % trans-tilidine) in 2-propanol was concentrated in a rotary evaporator at 100 mbar such that a solution containing 27.2 % of tilidine was obtained. 147.5 g (1638 mmol) of oxalic acid and 59 g (3276 mmol) of water were added to this solution.

b) Isomerization reaction

The reaction mixture obtained in step a) was directed through a plug flow reactor where it was maintained at 140 °C for 13 minutes.

c) Water removal and crystallization

The main fraction (1185 mmol tilidine) was diluted with 970 g of 2-propanol and then 679 g of solvent were distilled off at 100 mbar. After cooling down to 50 °C, 0.1 g of trans-tilidine oxalate were added, the mixture was cooled to 20 °C and then filtered. The filtration provided a filter cake and a mother liquor. The filter cake was washed twice with 368 g of 2-propanol. The washed filter cake was then dried in a vacuum drying oven at 50 °C.

194 g of trans-tilidine oxalate were obtained (498 mmol, purity: 93.2 % by weight, yield: 42 % based on the main fraction). The product comprised 0.04 % by weight of 2-propyl 2-(dimethylamino)-2-phenylcyclohex-3-ene-1-carboxylate side product.

Example 6

a) Provision of reaction mixture

To the combined mother and wash liquors obtained in example 5, step c) (1981 g, 596 mmol tilidine, 430 mmol

oxalic acid) 15.3 g of oxalic acid (166 mmol) were added. 1398 g of solvent were distilled off and then 54 g of water (2980 mmol) were added.

b) Isomerization reaction

The reaction mixture obtained in step a) was directed through a plug flow reactor where it was maintained at 140 °C for 13 minutes.

c) Water removal and crystallization

The main fraction (471 mmol tilidine) was diluted with 904 g of 2-propanol and then 831 g of solvent were distilled off at 100 mbar. After cooling down to 30 °C, 0.1 g of trans-tilidine oxalate were added, the mixture was cooled to 0 °C and then filtered. The filtration provided a filter cake and a mother liquor. The filter cake was washed twice with 236 g of 2-propanol. The washed filter cake was then dried in a vacuum drying oven at 50 °C.

92 g of trans-tilidine oxalate were obtained (225 mmol, purity: 88.8 % by weight, yield: 48 % based on the main fraction). The product comprised 0.101 % by weight of 2-propyl 2-(dimethylamino)-1-phenylcyclohex-3-ene-1-carboxylate side product.

The trans-tildine oxalate obtained in examples 1 and 2 comprised only very little side product that is difficult to separate, whereas the content of such side product was much higher in the product of comparative examples 3 and 4. The process according to the invention does therefore yield trans-tilidine dicarboxylic salt with only very little side product that is difficult to separate, i.e. trans-tilidine in a form that is essentially free of such side product.

In examples 5 and 6 a further increase in efficiency is obtained. The increased isomerization temperature allows for reducing the isomerization time from 9 hours to 13 minutes. Even though the isomerization is carried out at increased temperature, the obtained trans-tilidine dicarboxylic acid salt does only comprise very little side product that is difficult to separate.

## Claims

**1.** Tilidine isomerization and crystallization process, comprising

a) providing a reaction mixture comprising cis-tilidine, a dicarboxylic acid, water and an alcohol having 3 to 6 carbon atoms;
b) maintaining the reaction mixture at a temperature T of from 80 °C to 150 °C for a period of time t, T and t meeting the following condition:

$$500 \leq k^{(T-80)} \cdot t$$

wherein

k is 1,07738
T is the temperature in °C;
t is the time in minutes at the temperature T;

c) removing water from the reaction mixture and crystallizing a trans-tilidine dicarboxylic acid salt from the reaction mixture.

**2.** The process of claim 1, wherein the reaction mixture comprises 0.5 to 2 moles of dicarboxylic acid per mole of tilidine.

**3.** The process of claim 1 or 2, wherein the dicarboxylic acid is selected from aliphatic and aromatic dicarboxylic acids.

**4.** The process of any of the preceding claims, wherein the dicarboxylic acid is oxalic acid.

**5.** The process of any of the preceding claims, wherein the reaction mixture comprises 1 to 10 moles of water per mole of tilidine.

6. The process of any of the preceding claims, wherein the alcohol is selected from 2-propanol, 2-butanol, tert-butanol, 2-pentanol, 3-pentanol, 2-methyl-2-butanol, 2,2-dimethyl-1-propanol, 2-hexanol, 2-methyl-2-pentanol, 3-hexanol, 3-methyl-3-pentanol, cyclohexanol and their mixtures.

7. The process of any of the preceding claims, wherein the alcohol is selected from 2-propanol, 2-butanol, tert-butanol, 2-pentanol and their mixtures.

8. The process of any of the preceding claims, wherein the crystallization comprises cooling the reaction mixture to 25 °C or less.

9. The process of any of the preceding claims, wherein the crystallized trans-tilidine dicarboxylic acid salt is separated from the mother liquor by filtration or centrifugation.

10. The process of claim 9, wherein the mother liquor is recycled into step a), optionally after concentration.

11. The process of any of the preceding claims, wherein the removal of water is achieved by azeotropic distillation.

12. The process of claim 11, wherein the reaction mixture is diluted with alcohol having 3 to 6 carbon atoms before the water is removed by azeotropic distillation.

13. The process of any of the preceding claims, wherein step b) is carried out continuously in a plug flow type reactor.

## Patentansprüche

1. Tilidin-Isomerisierungs- und Kristallisationsverfahren, umfassend

a) Bereitstellen einer Reaktionsmischung umfassend cis-Tilidin, eine Dicarbonsäure, und ein Alkohol mit 3 bis 6 Kohlenstoffatomen;
b) Halten der Reaktionsmischung bei einer Temperatur T von 80 °C bis 150 °C für einen Zeitraum t, T und t, der die folgende Bedingung erfüllt:

•

$$500 \leq k\ (T\text{-}80) \cdot t$$

Worin

k ist 1,07738
T ist die Temperatur in °C;
15 t ist die Zeit in Minuten bei der Temperatur T;

c) Entfernen von Wasser aus der Reaktionsmischung und Kristallisieren eines Trans-Tilidins Dicarbonsäuresalz aus der Reaktionsmischung.

2. Verfahren nach Anspruch 1, wobei das Reaktionsgemisch 0,5 bis 2 Mole umfaßt Dicarbonsäure pro Mol Tilidin.

3. Verfahren nach Anspruch 1 oder 2, wobei die Dicarbonsäure ausgewählt ist aus aliphatischen und aromatischen Dicarbonsäuren.

4. Verfahren nach einem der vorhergehenden Ansprüche, wobei die Dicarbonsäure Oxalsäure ist.

5. Verfahren nach einem der vorhergehenden Ansprüche, wobei das Reaktionsgemisch 30 Prise 1 bis 10 Mol Wasser pro Mol Tilidin.

6. Verfahren nach einem der vorhergehenden Ansprüche, wobei der Alkohol ausgewählt ist aus 2-Propanol, 2-Butanol,

tert-Butanol, 2-Pentanol, 3-Pentanol, 2-Methyl-2-Butanol, 2,2-Dimethyl-1-propanol, 2-Hexanol, 2-Methyl-2-pentanol, 3-Hexanol, 3-Methyl-3-Pentanol, Cyclohexanol und ihre Gemische.

**7.** Verfahren nach einem der vorhergehenden Ansprüche, wobei der Alkohol ausgewählt ist aus 2-Propanol, 2-Butanol, tert-Butanol, 2-Pentanol und deren Gemische.

**8.** Verfahren nach einem der vorhergehenden Ansprüche, wobei die Kristallisation umfasst Abkühlen der Reaktionsmischung auf 25 °C oder weniger.

**9.** Verfahren nach einem der vorhergehenden Ansprüche, wobei das kristallisierte Trans-Tilidin Dicarbonsäuresalz wird durch Filtration oder Zentrifugation von der Mutterlauge abgetrennt.

**10.** Verfahren nach Anspruch 9, wobei die Mutterlauge in Schritt a) zurückgeführt wird, wobei nach der Konzentration.

**11.** Verfahren nach einem der vorhergehenden Ansprüche, wobei die Entfernung von Wasser durch azeotrope Destillation erfolgt.

**12.** Verfahren nach Anspruch 11, wobei das Reaktionsgemisch mit einem Alkohol enthaltend 3 bis 6 Kohlenstoffatome verdünnt ist bevor das Wasser durch azeotrope Destillation entfernt wird.

**13.** Verfahren nach einem der vorhergehenden Ansprüche, wobei Schritt b) kontinuierlich in einem Pfropfenströmungsreaktor durchgeführt wird.

## Revendications

**1.** Procédé d'isomérisation et de cristallisation de la tilidine, le procédé comprenant

a) fournir un mélange de réaction comprenant de la cis-tilidine, un acide dicarboxylique, ter et un alcool ayant 3 à 6 atomes de carbone;
b) maintenir le mélange de réaction à une température T de 80 °C à 150 °C pendant une période t, T et t satisfaisant aux conditions suivantes:

$$500 \leq k^{(T-80)}\,t$$

où

k est 1,07738
T est la température en °C;
t est le temps en minutes à la température T;

c) éliminer l'eau du mélange de réaction et cristalliser une trans-tilidine sel d'une acide dicarboxylique du mélange de réaction.

**2.** Procédé selon la revendication 1, dans lequel le mélange réactionnel comprend 0.5 à 2 moles d'une acide dicarboxylique par mole de tilidine.

**3.** Procédé selon la revendication 1 ou 2, dans lequel l'acide dicarboxylique est choisi parmi les acides dicarboxyliques aliphatiques et aromatiques.

**4.** Procédé selon l'une quelconque des revendications précédentes, dans lequel l'acide dicarboxylique est l'acide oxalique.

**5.** Procédé selon l'une quelconque des revendications précédentes, dans lequel le mélange de réaction comprend 1 à 10 moles d'eau par mole de tilidine.

6. Procédé selon l'une quelconque des revendications précédentes, dans lequel l'alcool est choisi parmi le 2-propanol, le 2-butanol, le tert-butanol, le 2-pentanol, le 3-pentanol, le 2-méthyl-2-butanol, le 2,2-diméthyl-1-propanol, le 2-hexanol, le 2-méthyl-2-pentanol, le 3-hexanol, le 3-méthyl-3- pentanol, le cyclohexanol et leurs mélanges.

7. Procédé selon l'une quelconque des revendications précédentes, dans lequel l'alcool est choisi parmi le 2-propanol, le 2-butanol, le tert-butanol, le 2-pentanol et leurs mélanges.

8. Procédé selon l'une quelconque des revendications précédentes, dans lequel la cristallisation comprend refroidir le mélange de réaction à 25 °C ou moins.

9. Procédé selon l'une quelconque des revendications précédentes, dans lequel la trans-tilidine cristallisée Le sel d'acide dicarboxylique est séparé de la liqueur mère par filtration ou centrifugation.

10. Procédé selon la revendication 9, dans lequel la liqueur mère est recyclée à l'étape a), option allié après la concentration.

11. Procédé selon l'une quelconque des revendications précédentes, dans lequel l'élimination de l'eau est obtenue par distillation azéotropique.

12. Procédé selon la revendication 11, dans lequel le mélange réactionnel est dilué avec de l'alcool où 3 à 6 atomes de carbone avant l'élimination de l'eau par distillation azéotropique.

13. Procédé selon l'une quelconque des revendications précédentes, dans lequel l'étape b) est réalisée en continu dans un réacteur du type à écoulement piston.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 1518959
- DE 1618476
- DE 1618482
- DE 1668156
- DE 1768704
- DE 1811808
- DE 1923619
- DE 1923620
- GB 1120186 A, Warner-Lambert
- US 3637806 A
- DE 1951587
- GB 1226318 A


### Non-patent literature cited in the description


- **K.-O. VOLLMER ; F. W. KOSS.** *Arzneimittelforschung,* 1970, vol. 20, 990
- **G. SATZINGER.** *Liebigs Ann. Chem.,* 1969, vol. 728, 64
- **G. SATZINGER.** *Liebigs Ann. Chem.,* 1972, vol. 758, 43
- **G. SATZINGER.** *Liebigs Ann. Chem.,* 1972, vol. 758, 65